(19) Europäisches Patentamt / European Patent Office / Office européen des brevets

(11) **EP 2 975 949 B2**

(12) **NEW EUROPEAN PATENT SPECIFICATION**
After opposition procedure

(45) Date of publication and mention
of the opposition decision:
**08.03.2023 Bulletin 2023/10**

(45) Mention of the grant of the patent:
**19.09.2018 Bulletin 2018/38**

(21) Application number: **14710585.2**

(22) Date of filing: **18.03.2014**

(51) International Patent Classification (IPC):
**A23J 1/14** $^{(2006.01)}$

(52) Cooperative Patent Classification (CPC):
**A23J 1/14**

(86) International application number:
**PCT/EP2014/055409**

(87) International publication number:
**WO 2014/147068 (25.09.2014 Gazette 2014/39)**

(54) **METHOD FOR PROTEIN EXTRACTION FROM OIL SEED**

VERFAHREN ZUR EXTRAKTION VON PROTEINEN AUS ÖLSAAT

PROCÉDÉ D'EXTRACTION DE PROTÉINES DE GRAINES OLÉAGINEUSES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.03.2013 EP 13159752**

(43) Date of publication of application:
**27.01.2016 Bulletin 2016/04**

(73) Proprietor: **DSM IP Assets B.V.
6411 TE Heerlen (NL)**

(72) Inventors:
• **JARAMILLO FREYDELL, Gabriel Esteban
NL-6100 AA Echt (NL)**
• **VERKAIK, Antonius Gosen Maria
NL-6100 AA Echt (NL)**
• **SMOLDERS, Gerardus Johannes Franciscus
NL-6100 AA Echt (NL)**

(74) Representative: **DSM Intellectual Property
P.O. Box 4
6100 AA Echt (NL)**

(56) References cited:
WO-A1-2013/013949    WO-A2-2006/047445
GB-A- 2 461 093    US-A- 6 005 076
US-A1- 2010 173 064    US-A1- 2012 252 065
US-A1- 2012 252 065

• **ROSENTHAL ET AL.: TRANS ICHEME, PART C, vol. 76, 1998, pages 224-230, XP022525174, cited in the application**
• **ROSENTHAL ET AL.: ENZYME AND MICROBIAL TECHNOLOGY, vol. 19, 1996, pages 402-420, XP002590775, cited in the application**
• **JOSHI J B ET AL: "Continuous counter-current two-phase aqueous extraction", BIOSEPARATION, KLUWER ACADEMIC PUBLISHERS, DORDRECHT, NL, vol. 1, no. 3-4, 1 January 1990 (1990-01-01), pages 311-324, XP009178440, ISSN: 0923-179X**

Remarks:
The file contains technical information submitted after the application was filed and not included in this specification

**Description**

**Field of the invention**

The present invention relates to the selective extraction of proteins over oil from oil seed meal, preferably from cold pressed oilseed meal, for the purpose of producing an intermediate aqueous protein solution which is suitable for preparing protein isolates composed of native proteins.

**Background of the invention**

Oilseeds (e.g. sunflower, rapeseed/canola, mustard seed, corn seed, flax seed) and soybeans are in general an excellent source of not only edible oils but also proteins. Canola for example is one of the largest oil seed crops in the world, considered to be the third most abundant source of edible oil.

Traditionally for materials having relatively high oil content (>35% on dry matter, rapeseed approximately 40%) a combination of mechanical pressing and solvent extraction is used for an efficient extraction of the oil [Rosenthal et al, Enzyme and Microbial Technology 19 (1996) 402-420]. After the oil is extracted, the pressed material is heat treated to remove the solvent, resulting in a cake with an oil and protein content of 1-5% and 40-50% of the dry matter, respectively. Although the cake has relative high protein content, the quality of the proteins present has been significantly reduced by the harsh conditions (i.e., elevated temperature, solvents) employed during the oil extraction. These harsh conditions lead to protein denaturation, consequently negatively affecting the functional properties of the purified proteins [Khattab et al, LWT - Food Science and Technology 42 (2009) 1119-1124], thereby decreasing their value. The awareness that these oil extraction conditions are detrimental for the quality of the proteins is one of the factors bolstering the improvement of the cold pressing technology. During cold pressing, no solvents are used and the oil is pressed out under mild conditions, resulting in better quality oil and an oilseed pressed meal of higher quality.

This meal has a relatively high oil content (typically > 8%, for example >10%, in dry matter basis) and is an excellent source of proteins with preserved functionality. These proteins can be readily extracted from the meal by for instance an aqueous extraction [Rosenthal et al, Enzyme and Microbial Technology 19 (1996) 402-420, Rosenthal et al, Trans IChemE, Part C, 76 (1998) 224-230 and Lawhon et al, Journal of Food Science 46 (1981) 912-916]. One of the biggest challenges of this type of processes is that during extraction proteins and oil are extracted concomitantly [Rosenthal et al, Trans IChemE, Part C, 76 (1998) 224-230]. This leads to an extract containing a significant amount of oil, present in most cases partly as a stable emulsion making its removal quite difficult.

The present invention addresses this challenge by showing that a protein rich practically fat free extract can be produced by selectively extracting the proteins using gentle extraction methods. As it will be explained in more detail later on, gentle extraction methods are extraction methods that generate minimum to no shear during extraction, which is important for the selective extraction of the proteins essentially without the extraction of the fat.

The method of the invention is particularly useful for extracting proteins from oil seed meals having an oil content of at least 8% on dry matter basis.

US 2012/0252065 describes an aqueous process for the preparation of a protein isolate and a hydrolyzed protein concentrate from an oil seed meal. The oil seed meal is mixed with an aqueous solvent to form a slurry. According to Figure 4A, the used mixing time is 1 hour resulting in undesired fat to protein ratios in the intermediate protein aqueous protein solution.

US 6,005,076 describes a method for preparing a protein isolate which comprises multiple steps. Protein extraction is performed by mixing oil seed meal with a salt solution and mixing/stirring at 76 rpm. According to the specification, protein extraction has the additional effect of solubilizing certain fats in the canola meal, which results in the fats being present in the aqueous phase. One of the other steps is the removal of fat from the aqueous phase.

US 2010/0173064 describes a protein isolate consisting predominantly of 2S canola protein which is obtained in a fragmentation process including agitation.

WO 2013/013949 describes a process to isolate protein from the meal or oil cake, for example in a stirred tank.

Klockeman et al (1997, J. Agric. Food Chem, 45, 3867-3870, Isolation and characterization of defatted canola meal protein) describe extraction of protein from commercial hexane defatted canola meal by performing agitation at 180-200 rpm for 60 minutes.

WO 95/27406 describes a method for producing a dietetic soy based product by suspending defatted (fat free) soy based material in water and subjecting the suspension to enzymatic treatment.

GB 2461093 describes a process for preparing a vegetable protein concentrate from oleaginous vegetable material comprising using an apolar solvent (such as hexane) and using ethanol.

GB 1 502 959 describes a process for treating de-oiled oleaginous seed material to produce a protein concentrate and an extract solution by using an organic solvent.

Rosenthal et al (1998, Trans iChemE, vol 76, part C, Simultaneous aqueous extraction of oil and protein from soybean: mechanisms for process design) investigate aqueous extraction of oil and protein from soybean flour. Protein and oil extraction yield were shown to be closely related. Figure 10 shows the effect of agitation speed on oil and protein extraction from soybean flour.

## Description of the Figures

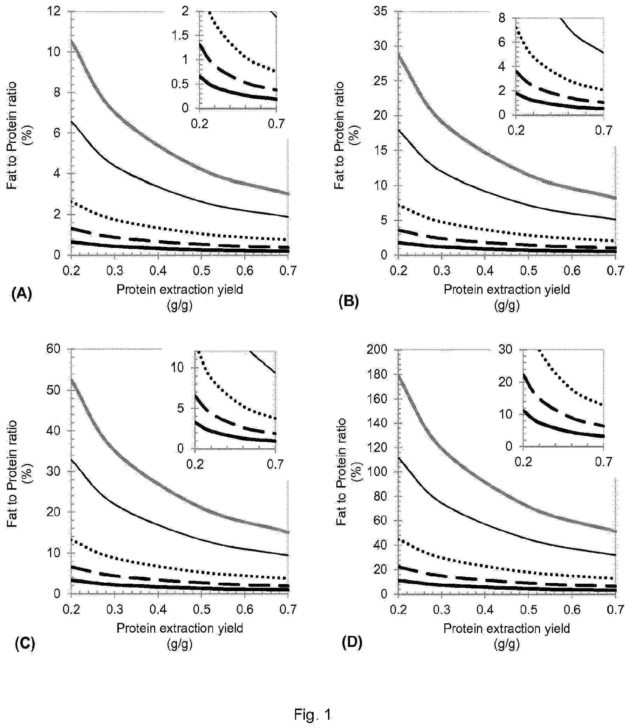

Figure 1: Fat to protein ratio as a function of the protein and oil extraction yields.

(A) Defatted rapeseed cake with an oil content of 1% on dry weight basis (DWB), a protein content of 38% on DWB, and a dry matter (DM) of 92%;
(B) Defatted rapeseed cake with an oil content of 2.6% on DWB, a protein content of 36.2% on DWB, and a DM of 89.2%[Shahidi F, Canola and Rapeseed: Production, Chemistry, Nutrition and Processing Technology. 1990 Van Nostrand Reinhold, ISBN 0-442-00295-5];
(C) Defatted rapeseed cake with an oil content of 5% on DWB, a protein content of 38% on DWB, and a DM of 92%;
(D) Cold pressed rapeseed cake with an oil content of 5% on DWB, a protein content of 38% on DWB, and a DM of 92%.

Solid thick line: oil extraction yield 5%;
Dashed line: oil extraction yield 10%;
Dotted line: Oil extraction yield 20%;
Solid thin line: Oil extraction yield 50%;
Grey line: Oil extraction yield 80%.

Insets: zoomed in regions in the low values of the fat to protein ratio. The fat to protein ratio was calculated using Equation (3) (see experimental part).
Figure 2: Fat to protein ratio during time in a stirred vessel (see also example 3)
Figure 3: Schematic extraction columns

## Summary of the invention

In one of its embodiments, the invention provides a method for producing from oil seed meal an intermediate aqueous protein solution having a fat to protein ratio of at least 1:12 comprising subjecting oil seed meal having an oil content of at least 8% on dry matter basis to gravity induced solid-liquid extraction and optionally collecting the resulting intermediate aqueous protein solution wherein said gravity induced solid-liquid extraction comprises percolation.

## Detailed description of the invention

The present invention shows that a protein rich, practically fat free, extract can be produced from a pressed oil seed meal by selectively extracting the proteins using gentle extraction methods. Gentle extraction methods are extraction methods that generate minimum to no shear during extraction, which is important for the selective extraction of the proteins essentially without the extraction of the fat.

Oil seed protein isolates are produced by a multistep process. In general such a process for producing a protein isolate from oil seed comprises:

- obtaining oil seed
- screening, cleaning and optionally dehulling of oil seed
- at least partly (solvent or cold) extracting oil from the dehulled oil seed resulting in an oil product and an at least partly defatted oil seed meal
- separating said oil product from said partly defatted oil seed meal
- extracting said partly defatted oil seed meal to cause solubilization of the protein in said partly defatted oil seed meal which typically also solubilizes (part of) the remaining fat in said partly defatted oil seed meal
- separating the aqueous protein solution from residual oil seed meal to obtain an intermediate aqueous protein solution; as outlined above such a solution typically comprises co-extracted fats and as a result the fat to protein ratio is, at this phase of the overall production method, typically well above 0.5%.
- subjecting the resulting intermediate aqueous protein solution to steps such as:

- a pigment removal step and/or fat removal step (the order of these steps can be reversed)

- concentrating the obtained protein solution to increase the protein concentration
- further fat removal step
- drying the concentrated protein solution to obtain an oil seed protein isolate; alternatively, precipitating the protein in solution by altering factors including temperature, pH, ionic strength, salt concentration and/or solvents, allowing the settling of the protein precipitate, separating precipitated proteins from residual aqueous phase and drying of proteins to obtain an oil seed protein isolate.

In case of rapeseed, dilution is typically used to precipitate proteins, for example dilution with chilled water to decrease the solubility of all components or dilution is done to decrease the ionic strength to a minimum, decreasing the solubility of those components soluble at relatively high ionic strength.

Alternatively, concentrated protein is washed and dried directly (i.e. without precipitation).

Depending on the exact conditions used and depending on the source material (i.e. the particular oil seed meal) used not all steps need to be performed and some of the steps might be performed in a different order. Furthermore not all steps need to be performed at once and in the same facility. Typically the oil is extracted from the oil seed in one facility (oil manufacturer) and the protein extraction from the oil seed meal/cake is performed at another facility.

To avoid any confusion, the present invention relates to a method for obtaining an intermediate aqueous protein solution (alternatively: protein extract stream or protein extract, the terms are used interchangeably herein), i.e. a protein solution which is the direct resulting product after aqueous extraction of a(n) (partly defatted) oil seed meal. Depending on the type of gentle extraction and depending on whether or not this gentle extraction is performed in a batch or continuous way, it might be necessary to perform a solid/liquid extraction before the intermediate aqueous protein solution is obtained. Alternatively phrased, the intermediate aqueous protein solution is the protein solution comprising the solubilized proteins from the (partly defatted) oil seed meal and which is the direct result of the extraction. The intermediate aqueous protein solution is the protein fraction which is not yet subjected to concentration, precipitation and/or drying. Alternatively phrased, an intermediate aqueous protein solution is the liquid phase as present in the solid/liquid mixture at the end of the extraction step. To avoid any misunderstanding, the intermediate aqueous protein solution is not a protein solution which is the result of a defatting, de-oiling or decreaming step) for example such as an extract obtained after centrifugation). An intermediate aqueous protein solution as described herein is different from a slurry as produced in prior art protein extraction methods. The term "slurry" typically refers to oil seed meal that has been extensively mixed (or agitated) with an aqueous solvent to form a liquid containing dissolved protein and a suspension of protein, oil and optionally fiber and anti-nutritional compounds, in the liquid.

The presence of fat in a protein intermediate solution is undesired because this leads to a protein extract which is partly present as a stable emulsion. Furthermore, the presence of fat may lead to protein product loss during further separation of proteins and fat/emulsions. Presence of (high levels) of fat in an intermediate protein solution could result in a relative high fat content in the end-product (the protein isolate) which is undesirable as well. The present invention addresses these problems by showing that a protein rich practically fat free extract can be produced by selectively extracting the protein using gentle protein extraction methods.

The present invention provides an aqeous method for producing from oil seed meal an intermediate aqueous protein solution having a fat to protein of at least 1:12 comprising subjecting oil seed meal having an oil content of at least 8% on dry matter basis to aqueous extraction under minimal shear conditions, gravity induced solid-liquid extraction, and optionally collecting the resulting intermediate aqueous protein solution wherein said gravity induced solid-liquid extraction comprises percolation.

Alternatively phrased, the present invention provides a method for selectively extracting proteins (over oil) from oil seed meal having an oil content of at least 8% on dry matter basis comprising subjecting said meal to aqueous extraction under minimal shear conditions, gravity induced solid-liquid extraction wherein said gravity induced solid-liquid extraction percolation. Preferably, the present invention provides a method for selectively extracting proteins (over oil) from oil seed meal having an oil content of at least 8% on dry mater basis, to obtain an intermediate aqueous protein solution having a fat to protein ratio of at least 1:12, comprising subjecting said meal to aqueous extraction under minimal shear conditions, gravity induced solid-liquid extraction wherein said gravity induced solid-liquid extraction comprises percolation.

The method of the invention can be performed on any oil seed or soybean meal. Examples of oil seed meals are rapeseed, flax, linola, sunflower or mustard seed meals. The invention as described herein is more particularly related to rapeseed meal. Rapeseed is also known as rape, oilseed rape, rapa, rappi, rapeseed and canola. In a preferred embodiment, the invention provides an aqueous method for producing from oil rapeseed meal an intermediate aqueous rapeseed protein solution having a fat to protein ratio of at least 1:12 comprising subjecting oil rapeseed meal having an oil content of at least 8% on dry mater basis to aqueous extraction under minimal shear conditions (preferably gravity induced solid-liquid extraction) and optionally collecting the resulting intermediate aqueous rapeseed protein solution wherein said gravity induced solid-liquid extraction comprises percolation.

The used oil seed meal may comprise naturally occurring protein or may comprise genetically modified oil seed, i.e. oil seed in which at least one protein is genetically modified.

The oil seed meal (or alternatively the oil seed cake; the terms are used interchangeably herein) may be any meal (or cake) resulting from the removal of oil from the seeds. The oil seed meal may comprise varying levels of remaining oil. For example, the oil seed meal is the result of hexane extraction and comprises typically between 1-5% oil on dry weight basis (DWB). Alternatively, the oil seed meal is the result of a cold pressing method and typically comprises between 10-25% on DWB or typically comprises at least 8% oil on dry matter basis. In a preferred embodiment the oil seed meal has an oil content of at least 8% or at least 10% and hence the invention provides an aqueous method for producing from oil seed meal, having an oil content of at least 8% (or 10%) (preferably in the range of 8-25% or 10-25% DWB) on dry matter basis, an intermediate aqueous oil seed protein solution having a fat to protein ratio of at least 1:12 comprising subjecting oil seed meal having an oil content of at least 8% to aqueous extraction under minimal shear conditions (preferably gravity induced solid-liquid extraction) and optionally collecting the resulting intermediate aqueous oil seed protein solution wherein said gravity induced solid-liquid extraction comprises percolation. In an even more preferred embodiment, said oil seed meal is oil rapeseed meal having an oil content of at least 8% (or at least 10%) on dry matter basis and the invention thus also provides a method for producing from oil rapeseed meal, having an oil content of at least 8% (or 10%) on dry matter basis, an intermediate aqueous rapeseed protein solution having a fat to protein ratio of at least 1:12 comprising subjecting said oil rapeseed meal to aqueous extraction under minimal shear conditions (preferably gravity induced solid-liquid extraction) and optionally collecting the resulting intermediate aqueous rapeseed protein solution wherein said gravity induced solid-liquid extraction comprises percolation.

An example of an oil seed meal having an oil content of at least 8 or of at least 10% on dry matter basis is a cold pressed oil seed meal. The invention also provides an aqueous method for producing from cold pressed oil seed meal an intermediate aqueous protein solution having a fat to protein ratio of at least 1:12 comprising subjecting said cold pressed oil seed meal to aqueous extraction under minimal shear conditions (preferably gravity induced solid-liquid extraction) and optionally collecting the resulting intermediate aqueous protein solution wherein said gravity induced solid-liquid extraction comprises percolation. In an even more preferred embodiment, said cold pressed oil seed meal is cold pressed oil rapeseed meal and the invention thus also provides an aqueous method for producing from cold pressed oil rapeseed meal an intermediate aqueous protein solution having a fat to protein ratio of at least 1:12 comprising subjecting said cold pressed oil rapeseed meal to aqueous extraction under minimal shear conditions (preferably gravity induced solid-liquid extraction) and optionally collecting the resulting intermediate aqueous protein solution wherein said gravity induced solid-liquid extraction comprises percolation. The seeds from which the used oil seed meal is prepared can be hulled or can be subjected to a dehulling step (i.e. the hulls are removed from the oil seeds).

The fat to protein ratio in the intermediate aqueous protein solution is easily calculated as described in the experimental part herein. The experimental part also provides theoretical calculations of the fat to protein ratio in intermediate aqueous protein solutions as described in the prior art. The prior art extraction methods are not very selective, i.e. the extraction methods of the prior art co-extract fat during protein extraction, and hence it is believed that prior art fat to protein ratio in intermediate aqueous protein solutions are far below 1:12 (generally, the intermediate aqueous protein solutions of the prior art have a fat to protein ratio of around 1:3, see also the experimental part as described herein). The present invention provides an aqueous method to selectively extract protein from an oil seed meal and it is therefore possible to obtain a more favourable fat to protein ratio in an intermediate aqueous protein solution. The present invention relates to an aqueous method for obtaining an intermediate aqueous protein solution. Preferably, the invention relates to an aqueous method for obtaining an intermediate aqueous protein solution having a fat to protein ratio of at least 1:12. The phrase "fat to protein ratio of at least 1:12" should be read as a ratio in which the amount of protein is 12 or higher. More preferably, said fat protein ratio is at least 1:13, 1:14, 1:15 , 1:16, 1:17, 1:18, 1:19 or 1:20. More favourable fat to protein ratios in which the amount of protein is higher than 20 are also included herein. The invention thus provides an aqueous method for producing from (preferably cold pressed) oil seed meal (preferably rapeseed meal) an intermediate aqueous protein solution having fat to protein ratio of at least 1:12 or more preferably of at least 1:13, 1:14, 1:15, 1:16, 1:17, 1:18, 1:19 or 1:20 comprising subjecting oil seed meal having an oil content of at least 8% on dry matter basis to aqueous extraction under minimal shear conditions, gravity induced solid-liquid extraction and optionally collecting the resulting intermediate aqueous protein solution wherein said gravity induced solid-liquid extraction comprises percolation.

The phrase "subjecting oil seed meal to aqueous extraction" as used herein typically refers to a step of bringing an aqueous solution in contact with an oil seed meal. This is, for example, done by loading the oil seed meal into a container (for example a column) equipped with perforated supports used to keep the oil seed meal inside, consequently creating a fixed bed of oilseed meal. Thereafter, the aqueous solution is pumped throught the fixed bed, thereby contacting both solid and liquid phases and initiating the extraction process under minimal shear conditions. Alternatively, the oil seed meal and the aqueous solution are added simultaneously to a tube or column. As a result, the oil seed meal is soaked into the aqueous solution and the meal is divided into (rather uniform) particles. In yet another alternative, the aqueous solution is put into a tube or column and the oil seed meal is added thereto. Again, the oil seed meal is soaked and the meal is divided into (rather uniform) particles.

The term "extraction under minimal shear conditions" is used herein to describe how the extraction should be performed. Preferably, the extraction of oil seed meal is performed such that the physical forces on the oil seed meal are gentle/mild. As described by Rosenthal et al [Trans IChemE, Part C, 76 (1998) 224-230 and Lawhon et al, Journal of Food Science 46 (1981) 912-916] one of the biggest challenges during proteins extraction is that proteins and oil are extracted concomitantly. This leads to an, undesired, extract containing a significant amount of oil, present in most cases as a stable emulsion making its removal quite difficult. Moreover, the presence of fat could result in an overall reduced protein yield or in a protein product with relative high fat content. These are all undesired results. The present invention shows that proteins can be selectively extracted (over fat) by using minimal shear conditions. Such minimal shear conditions can for example be obtained by performing the extraction in a stirred vessel operating at low rpm. Agitation for 30-120 minutes in (large scale) stirred vessels is commonly used in the field of protein extraction from oil seed meal. Protein extraction from an oil seed meal on laboratory scale is typically performed using mechanical stirring. Independent of the scale size, prior art protein extractions from oil seed meal/cake are performed under relative high shear conditions and as a result the fat co-extracts with the proteins from the oil seed meal.

The use of relative low rpm values (in a stirred vessel) already lead to a selective extraction of proteins from oil seed meal. More impressive (and surprising) and more industrial relevant results are obtained when extraction of the proteins from oil seed meal is performed under non mechanical mixing conditions or alternatively phrased, when the solids of the oil seed meal are not exposed to mechanical stress. In one of its embodiments, the invention provides an aqueous method for producing from oil seed meal an intermediate aqueous protein solution having a fat to protein ratio of at least 1:12 comprising subjecting oil seed meal having an oil content of at least 8% on dry matter basis to aqueous extraction under minimal shear conditions and optionally collecting the resulting intermediate aqueous protein solution, wherein said extraction under minimal shear conditions comprises extraction under non mechanical mixing conditions. In relation to protein extraction from oil seed meal, mechanical mixing conditions are conditions in which the solids of the oil seed meal as well as the aqueous extraction solution are constantly brought into agitation. As a consequence, the solids are disrupted by the force of the agitation/stirring. The present invention shows that when the solids of the oil seed meal are not exposed to mechanical stress, proteins can be selectively extracted from oil seed meal. The invention thus provides an aqueous method for producing from oil seed meal an intermediate aqueous oil seed protein solution having a fat to protein ratio of at least 1:12 comprising subjecting oil seed meal having an oil content of at least 8% on dry matter basis to aqueous gravity induced solid-liquid extraction and optionally collecting the resulting intermediate aqueous oil seed protein solution wherein said gravity induced solid-liquid extraction comprises percolation, wherein said extraction under minimal shear conditions comprises extraction wherein the solids (of the oil seed meal) are not or hardly not mechanically agitated. The term "hardly not mechanically agitated" is used to make clear that during extraction some kind of movement of the solids by a mechanical device is allowed as long as it does not or hardly not lead to disruption of the solids. For example, the use of low shear pump like a peristaltic pump or a monopump or a similar low shear pump known by the skilled person is an example of a device which does not or hardly not lead to disruption of the solids. Without being bound by it, it is currently thought that by extracting proteins from oil seed meal using gentle extraction methods, the solids are not disrupted and the oils are thus not released from disrupted solids resulting in selective protein extraction.

In a preferred embodiment, the phrase "extraction under minimal shear conditions" comprises gravity induced solid-liquid extraction, i.e. extraction in which the solids (and the liquid) essentially only move due to gravity or phrased differently, the solids essentially only move in a vertical way. Gravity induced solid-liquid extraction does not use agitation and/or stirring during extraction. Preferred embodiments of "gravity induced solid-liquid extraction" are

- gravity induced mass flow solid-liquid extraction, or
- co-current gravity induced solid-liquid extraction, or
- co-current gravity induced mass flow solid-liquid extraction, or
- counter-current gravity induced solid-liquid extraction, or
- counter-current induced mass flow solid-liquid extraction

During co-current solid-liquid extraction, the solids and liquids move into the same direction and during counter-current solid-liquid extraction the solids and liquids move into opposite direction in which case the solids only move due to gravity.

Gravity induced solid-liquid extraction should be understood to refer to "essentially only" gravity induced solid-liquid extraction, i.e. not ruling out that a very minor part of the extraction process includes a non-gravity induced step.

Gravity induced solid-liquid extraction can be performed in a column or tube with a suitable diameter which can be easily determined by the skilled person. In case the length of the column or tube is sufficient long to allow the liquid and solids to contact (i.e. resulting in a sufficiently long contact time of solids and liquids) to selectively extract the protein from the solids, the whole extraction process could be performed in one step, in which the solids and liquids are either added to the column or tube simultaneously or the liquid is added first and the solids are added somewhat later. Alternatively, a column or tube is used which is too short to extract sufficient protein in one step (i.e. insufficient contact time between solids and liquid) and the contact time is increased by recirculation in the same or a different column or tube. In this case, the solids and liquids are either added to the column or tube simultaneously or the liquid is added first and the solids are added somewhat later. After the solids have settled on the bottom of the column or tube, the content of the column or tube is removed with help of a low shear pump (such as a peristaltic pump or a monopump or a similar low shear pump known by the skilled person) and brought to the top of the same or another column or tube. Depending on the length and diameter of the column or tube, this process is repeated to obtain sufficient amount of contact time between the solids and the liquids. During settling on the bottom of the column or tube of the solids, the solids are in contact with the liquid and the proteins are extracted via percolation. In yet another alternative method, a column or tube is used which is too short to extract sufficient protein in one step (i.e. insufficient contact time between solids and liquid) and the contact time is increased by providing the column or tube with a bottom which can be opened above another column or tube allowing the solids again to contact the liquid and to settle. As outlined above, gravity induced solid-liquid extraction should be understood to refer to "essentially only" gravity induced solid-liquid extraction, i.e. not ruling at that a very minor part of the extraction process includes a non-gravity induced step. It is clear from the described process that one example of a non-gravity induced step is the use of a low shear pump to introduce the solid-liquid at the top of a column or tube. In a preferred embodiment, the invention provides an aqueous method for producing from oil seed meal an intermediate aqueous protein solution having a fat to protein ratio of at least 1:12, comprising subjecting oil seed meal having an oil content of at least 8% on dry matter basis to gravity induced solid-liquid extraction and subjecting said oil seed meal to a low shear device (preferably a low shear pump) and optionally collecting the resulting intermediate aqueous protein solution wherein said gravity induced solid-liquid extraction comprises percolation.

To extract protein from oil seed meal and to avoid disruption of the solids, the oil seed meal is brought into contact with an aqueous solution by using percolation. The invention therefore also provides an aqueous method for producing from oil seed meal an intermediate aqueous protein solution having a fat to protein ratio of at least 1:12 comprising subjecting oil seed meal having an oil content of at least 8% on dry mater basis to aqueous gravity induced solid-liquid extraction and optionally collecting the resulting intermediate aqueous protein solution, wherein said extraction under minimal shear conditions comprises percolation . In a preferred embodiment, said oil seed is rape seed. The invention thus provides an aqueous method for producing from oil rapeseed meal an intermediate aqueous rapeseed protein solution having a fat to protein ratio of at least 1:12 comprising subjecting oil rapeseed meal having an oil content of at least 8% on dry matter basis to aqueous gravity induced solid-liquid extraction and optionally collecting the resulting intermediate aqueous protein solution wherein said gravity induced solid-liquid extraction comprises percolation, wherein said extraction under minimal shear conditions comprises percolation. In yet another preferred embodiment, the oil rapeseed meal has an oil content of at least 10% on dry matter basis and the invention provides an aqueous method for producing from oil rapeseed meal an intermediate aqueous rapeseed protein solution having a fat to protein ratio of at least 1:12 comprising subjecting oil rapeseed meal having an oil content of at least 10% on dry matter basis to aqueous gravity induced solid-liquid extraction and optionally collecting the resulting intermediate aqueous protein solution, wherein said extraction under minimal shear conditions comprises percolation. Preferably, said oil rape seed meal is cold pressed oil seed meal. Immersion and percolation are well known techniques which are used in different technical fields, amongst others in the field of extraction of oil from seeds. In a percolation extraction, a solvent is for example distributed over a bed of oil seed flakes or cake, where it percolates down through the bed and exits the bed at the bottom through some type of supported filtering device such as a perforated plate or a mesh screen. The percolation process, also known as the continuous extraction process, is based upon the principle of uninterrupted wetting of extraction material. Here, solvent streams pass extraction material, allowing interrupted exchanges between this and the free-flowing solvent that extracts it. Maintenance of a constant solvent flow ensures that locally saturated solvent flows away and is replaced by non-saturated solvent. This process requires free extraction agent flow within the extraction material. In an immersion type of extraction, the oil seed meal is dispersed into a container previously filled with the aqueous phase. As the oil seed meal contacts the liquid it will swell and sink. During the sinking process, the hydrated oil seed comes in contact with fresh volumes of liquid, leading to the extraction of soluble components. In the bottom of the vessel, conveyor systems are used to transport the hydrated oil seed meal out of the container, while the liquid rich in protein exist by for example an overflow system.

As described above, the use of percolation and/or immersion was well known in the field of oil extraction. It is now shown by the present invention that these techniques, surprisingly, are very useful for selectively extracting proteins (compared to fat) from (partly defatted) oil seed meal. Preferred types of percolation are sprayed percolation, immersed percolation, solids dispersion or positive pressure percolation or a combination thereof. Yet another preferred type of percolation is recirculation percolation or multistage percolation. The invention therefore provides an aqueous method for producing from oil seed meal an intermediate aqueous protein solution having a fat to protein ratio of at least 1:12 comprising subjecting oil seed meal having an oil content of at least 8% on dry matter basis to aqueous gravity induced solid-liquid extraction and optionally collecting the resulting intermediate aqueous protein solution wherein said gravity induced solid-liquid extraction comprises percolation, wherein the oil seed meal is extracted with an aqueous solution by sprayed percolation, immersed percolation, solids dispersion or positive pressure percolation or recirculation percolation or multistage percolation or a combination thereof. In a preferred embodiment, said seed is rapeseed and the invention provides an aqeous method for producing from oil rapeseed meal an intermediate aqueous rapeseed protein solution having a fat to protein ratio of at least 1:12 comprising subjecting oil rapeseed meal having an oil content of at leat 8% on dry matter basis to aqueous gravity induced solid-liquid extraction and optionally collecting the resulting intermediate aqueous rapeseed protein solution wherein said gravity induced solid-liquid extraction comprises percolation, wherein the oil rapeseed meal is extracted with an aqueous solution by sprayed percolation, immersed percolation, solids dispersion or positive pressure percolation or recirculation percolation or multistage percolation or a combination thereof. More preferably, said oil seed meal has an oil content of at least 10% on dry matter basis and the invention provides an aqueous method for producing from oil rapeseed meal an intermediate aqueous rapeseed protein solution having a fat to protein ratio of at least 1:12 comprising subjecting oil rapeseed meal having an oil content of at least 10% to aqueous extraction under minimal shear conditions (preferably gravity induced solid-liquid extraction) and optionally collecting the resulting intermediate aqueous rapeseed protein solution wherein said gravity induced solid-liquid extraction comprises percolation, wherein the oil rapeseed meal is extracted with an aqueous solution by sprayed percolation, immersed percolation, solids dispersion or positive pressure percolation or recirculation percolation or multistage percolation or a combination thereof. Even more preferably, said oil seed meal is cold pressed oil seed meal. The oil seed meal can be derived from hulled or dehulled oil seeds.

Suitable equipment is for example described in Perry's Chemical Engineer's Handbook (more specific in the chapter titled "Leaching") and include - but is not limited to batch percolators, batch percolators under pressure (also known as diffusers), moving bed percolators (for example bucket elevator percolators, horizontal-belt conveyors, or Kennedy extractors), Pachua tanks, Bonotto extractor, Hildebrandt total -immersion extractor and screw-conveyor extractor.

The aqueous extraction used in the protein solubilization is preferably performed in the presence of a salt solution. All kinds of different salt solutions can be used, but preferebly the salt is sodium chloride. Another suitable salt is potassium chloride. Preferably, the salt solution has an ionic strength of at least about 0.10, more preferably at least about 0.15, to enable solubilization of significant quantities of protein to be effected. As the ionic strength of the salt solution increases, the degree of solubilization of protein in the source material initially increases until a maximum value is achieved. Any subsequent increase in ionic strength does not increase the total protein solubilized. The ionic strength of the food grade salt solution which causes maximum protein solubilzation varies depending on the salt concerned and the protein source chosen.

To avoid any misunderstanding, the term "aqueous extraction" or "aqueous solution" as used herein refers to an extraction or a solution which is free from organic solvents, such as methanol, propanol, iso-propanol, tetrahydrofuran or hexane since these solvents are not desirable as residues in a protein extract for human consumption. As a result, the intermediate aqueous protein solution as described herein does not or hardly not comprise denatured proteins.

In one of the embodiments, the invention provides an aqueous method for producing from oil seed meal an intermediate aqueous protein solution having a fat to protein ratio of at least 1:12 comprising subjecting oil seed meal having an oil content of at leats 8% on dry matter basis to aqueous gravity induced solid-liquid extraction and optionally collecting the resulting intermediate aqueous protein solution wherein said gravity induced solid-liquid extraction comprises percolation, wherein said aqueous extraction comprises an aqueous salt solution, preferably an aqueous salt solution with an ionic strength of at least 0.10, more preferably an aqueous salt solution with an ionic strength of at least 0.15. Preferred ranges are an aqueous salt solution with an ionic strength in the range of 0.10 to 0.8 or more preferably an aqueous salt solution with an ionic strength in the range of 0.15 to 0.8.

Suitable ratios of oil seed meal to aqueous solution are easily determined by the skilled person and are typically in the range of 1:5 to 1:12.

The solubilization is preferably performed at elevated temperatures, preferably above 5°C, generally up to about 65°C. The correct upper limit will be dictated by the denaturation temperature of the specific proteins to be extracted and can easily be determined and adjusted by the skilled person. For rapeseed the upper limit is approximately 65°C. In yet another preferred embodiment, the invention provides an aqueous method for producing from oil seed meal an intermediate aqueous protein solution having a fat to protein ratio of at least 1:12 comprising subjecting oil seed meal having an oil content of at least 8% on dry matter basis to aqueous gravity induced solid-liquid extraction and optionally collecting the resulting intermediate aqueous protein solution wherein said gravity induced solid-liquid extraction comprises percolation, wherein said aqueous extraction is performed at a temperature higher than 5°C, generally less than 65°C. A suitable temperature range is 5-65°C.

In yet another embodiment, the invention provides an aqueous method for producing from oil seed meal an intermediate aqueous protein solution having a fat to protein ratio of at least 1:12 comprising subjecting oil seed meal having an oil content of at least 8% on dry matter basis to aqueous gravity induced solid-liquid extraction and optionally collecting the resulting intermediate aqueous protein solution wherein said gravity induced solid-liquid extraction comprises percolation, wherein said aqueous extraction comprises an aqueous salt solution, preferably an aqueous salt solution with an ionic strength in the range of 0.10 to 0.80 (more preferably 0.15-0.8 and wherein said aqueous extraction is performed at a temperature in the range of 5-65°C.

The optimum pH value for maximum protein yield varies depending on the protein source material (e.g., solvent treated or cold pressed, de-hulled and non-dehulled) and are typically above pH 5.0 and below pH 8.0. pH values of about 6.0-7.0 are prefered for extracted rapeseed proteins The pH of the salt solution may be adjusted to any desired value within the range of about 5 to 8.0 for use in the extraction step by use of any convenient acid or alkali.

The optimum extraction time depends on the used experimental setting and can easily be determined by the skilled person. A suitable extraction time can for example be in the range of approximately 5-10 minutes or higher.

The aqueous extraction is preferably performed as a continuous process, enabling large scale throughput. However, batch or semi-continuous extraction is suitable as well. An example of a semi-continuous extraction is an extraction using multiple columns which are one after another filled, extracted and emptied.

Whether or not a step of "optionally collecting the resulting intermediate aqueous protein solution" is necessary, depends on the used gentle/mild extraction method. In case a stirred vessel (operating at low rpm value) is used, a solid/liquid separation is necessary to obtain the intermediate aqueous protein solution. Any solid/liquid separation technique can be used, such as employing vacuum filtration, followed by centrifugation and/or filtration to remove residual meal.

When using percolation extraction in a column with a fixed perforated bottom (as in example 2) the oil seed meal is retained in the extraction vessel while the extraction liquid typically flows through the oil seed meal bed. In continuous mode multiple oil seed meal fixed beds are used. These fix beds move in one direction while the liquid flow is switched to achieve a co-current or counter-current contact of the liquid and solid phase (oil seed meal fixed bed). A bed is exposed to liquid for a given amount of time and at the end of the extraction the vessel containing the spent bed is open, rinsed, and filled with fresh oil seed meal. The liquid flow is in the meantime constantly leaving the extraction unit, practically free of suspended solids. This liquid with a fat to protein ratio of at least 1:12, can be optionally clarified before being transferred to a collection vessel but typically does not to be collected. In case a column or tube is used, any of the above described solid-liquid separation techniques can be used. In case of recirculation percolation, it is also possible to use a final column or tube with some type of supported filtering device (for example a perforated plate or a mesh screen) through which the liquid can exist the column or tube.

A method of the invention is preferably performed on large scale basis, such as a method using at least 1 to 10 kg of rapeseed meal per hour or higher. Preferably, a method of the invention uses at least 10 kg oil seed meal per hour.

The invention is hereby illustrated with the following non-limiting examples

## Experimental part

### Materials and methods

**Protein content**

Protein content was determined by the Kjeldahl method according to AOAC Official Method 991.20 Nitrogen (Total) in Milk, using a conversion factor of 6.25 was used to determine the amount of protein (% (w/w)).

**Moisture content**

The moisture content was determined according to the: Food Chemical Codex, edition 7, General tests and assays, Appendix II, pages 1133 - 1134.

**Fat content**

The fat content was determined according to the method of AOCS 6th edition, Ce 1-62.

**Protein extraction yield**

The protein extraction yield is defined as follows

$$Y_{protein} = \frac{\text{Total amount of protein in the extract}}{\text{Total amount of protein in the starting material}} \qquad (1)$$

**Fat extraction yield**

The fat extraction yield is defined as follows

$$Y_{\text{fat}} = \frac{\text{Total amount of fat in the extract} + \text{Total amount of fat collected after centrifugation and sieving}}{\text{Total amount of fat in the starting material}}$$

$$(2)$$

**Fat to protein ratio**

The fat to protein ratio is defined as follows.

$$\frac{\text{Fat}}{\text{Protein}} = \frac{\text{Amount of fat extracted}}{\text{Amount of protein extracted}} = \frac{(RC)(DM)x_{\text{Fat,DWB}}Y_{\text{Fat,Extrac}}}{(RC)(DM)x_{\text{Pro,DWB}}Y_{\text{Pro,Extrac}}} = \frac{x_{\text{Fat,DWB}}Y_{\text{Fat,Extrac}}}{x_{\text{Pro,DWB}}Y_{\text{Pro,Extrac}}} 100\% \qquad (3)$$

where RC represents the amount of rapeseed cake, DM is the rapeseed cake dry matter content, $x_{\text{Pro,DWB}}$ is the rapeseed cake protein content as fraction of the dry matter, $Y_{\text{Pro,Extrac}}$ is the protein extraction yield, $x_{\text{Fat,DWB}}$ is the rapeseed cake fat content as fraction of the dry matter and $Y_{\text{Fat,Extrac}}$ is the fat extraction yield.

Alternatively, the fat to protein ratio is defined as:

Fat : protein -> 1 part fat per x parts of protein:

$$Ratio \frac{protein}{fat} = \frac{gram\ protein\ per\ gram\ dry\ material}{gram\ fat\ per\ gram\ dry\ material}$$

$$= \frac{\%\ protein\ on\ dry\ matter(\frac{w}{w})}{\%\ fat\ on\ dry\ matter\ (\frac{w}{w})} \quad (4)$$

**Fat to protein ratio after extraction: Deffated by pressing and solvent treatment rapeseed cake vs cold pressed rapeseed cake**

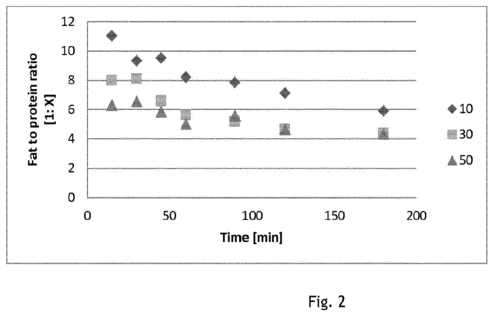

Figure 1 presents the fat to protein ratio as a function of the protein extraction yield, for defatted solvent treated rapeseed cake (oil content in %DWB of: 1% Fig1A; 2.6% Fig1B, and 5% Fig1C), and cold pressed cake (Fig 1D). Cold pressed cake was used to generate the examples presented in this invention. The typical oil content of a defatted solvent extracted rapeseed cake lies in the rage of 1-5% on dry weight basis (DWB), usually around 3% DWB [Shahidi F, Canola and Rapeseed: Production, Chemistry, Nutrition and Processing Technology. 1990 Van Nostrand Reinhold, ISBN 0-442-00295-5 and Rozenszain et al, WO 2012/135955A1]. In contrast, cold pressed rapeseed cake has an oil content in the range of 10-25% on DWB [Rozenszain et al, WO 2012/135955A1].

The dehulled cold pressed rapeseed cake used in our research has an oil and protein content of approximately 17% and 38%, respectively.

Figure 1A

From Figure 1A it can be seen that theoretically a fat to protein ratio in the range of 0.3% to 0.5% (1: 333 tot 1 : 200 as calculated with equation 4) can be attained using a defatted solvent treated rapeseed cake with an oil content of 1% on DWB. This relatively low values are however only plausible if the oil extraction yield falls in the range of 5-10% and the protein extraction yield lies in the range of 0.5-0.70. This situation is however very unlikely because protein aqueous extraction yield from defatted solvent treated meal has been reported to be in the range of 0.30-0.50, under non denaturing conditions [Klockeman et al, Journal of Agricultural and Food Chemistry,45 (1997) 3867-3870], and oil extraction yield in a stirred vessel system are unlikely to be lower than 10%. Hence, it is highly likely that the fat to protein ratio, obtained using defatted solvent treated meal with an oil content of 1% on DWB, is greater than 0.5%, most likely greater than 1% (Fig 1A).

Figure 1B and 1C

When using a 2.6% and 5% the fat to protein ratio is expected to be greater than 3% (Fig 1B), and 4% (Fig 1C), respectively; considering the aforementioned protein extraction and oil extraction yield constraints.

Figure 1D

In the case of cold press meal, the fat to protein ratio is expected to be greater than 5% (Fig 1D). When using percolation/gentle extraction the data shows that fat to protein ratios lower than 0.4% are feasible (see Table 2).

**Example 1 (prior art)**

This example describes the result of a conventional prior art aqueous extraction process. 600 grams of dehulled cold pressed rapeseed cake were added to 3 000 grams of an aqueous solution, containing no sodium chloride or sodium chloride at a concentration of 2% (w/w). A suspension was created by agitating the solution at 150 RPM. The extraction was performed at either 15 or 50°C. Mixing was done for 60 min. Thereafter, the suspension was fractionated by centrifugation at 4°C for 30 min. The centrifugation resulted in the separation of the depleted cake from the aqueous extract and the extracted fat. The aqueous extract and fat were separated by sieving, using a 150-250$\mu$m sieve. Table 1 presents the composition of the extract in terms of protein and fat content, as well as the fat and protein extraction yield. The protein and fat extraction yield were determined as described in the materials and methods section.

Table 1: Stirred vessel extraction: yields and extract composition

| Extraction conditions | | | | |
|---|---|---|---|---|
| Temperature (°C) | 50 | 50 | 15 | 15 |
| [NaCl] (%w/w) | 0 | 2 | 0 | 2 |
| Extraction yield | | | | |
| Protein (g/g) | 0.42 | 0.52 | [c)]0.35-0.36 | 0.46 |
| Fat (g/g) | 0.32 | 0.27 | [c)]0.25-0.30 | 0.29 |
| Fat to protein ratio | | | | |
| Fat/Protein [a)] (%) | 34.10 | 23.26 | 32.1-37.3 | 28.3 |
| Fat to protein [1:X][b)] | 2.93 | 4.29 | 3.11 | 3.5 |
| a) Estimated with Eq(3) b) calculated with equation 4 | | | | |

The skilled person is very well capable of calculating the fat to protein ratio expressed as [1:X] from the fat to protein ratio expressed as %. For example, if the fat/protein (%) is 34.10 this means that 0.341 fat is present and 1 protein is present. One needs to bring the 0.341 fat value to 1 by dividing with 0.341 and one needs to do the same for the protein value, i.e. divide 1 by 0.341, resulting in a protein value of 2.93.

From this experiment it can be concluded that: (a) the fat extraction yield is practically insensitive to the temperature and salt concentration, for a fixed mixing speed and stirrer type; and (b) the fat to protein ratio decreases as the extraction salt concentration increases, for a given extraction temperature. This decrease is reasonably explained by the increase in the protein extraction yield, which increases as a function of the salt concentration, for a given extraction temperature.

**Example 2 (process of the invention)**

This example illustrated the process of the invention

12 kg of dehulled cold pressed rapeseed cake was loaded into a 56 cm internal diameter jacketed stainless steel column. The bottom and top adaptors of the column were equipped with 5$\mu$m frits. Separately 150L of a 2%(w/w) sodium chloride solution were prepared. This solution was set to a temperature of 50°C and subsequently pumped through the column containing the dehulled cold pressed rapeseed cake. The temperature of the columns was adjusted before pumping the aqueous salt solution to a temperature of 50°C. The liquid was recirculated for a period of 2 hrs. Thereafter the clarified liquid was analysed for protein and fat. Table 2 presents the results obtained using different amounts of dehulled cold pressed rapeseed cake, with and without the recirculation of the aqueous salt solution. Experiments using 20g were done on a 5 cm internal diameter jacketed column and the experiment using 1 kg was done using a jacketed seitz filter.

Table 2: Percolation extraction of proteins from dehulled rapeseed at 50°C and a NaCl concentration of 2%(w/w)

| Yield (g/g) | | Extract composition (g/gDM) | | | | |
|---|---|---|---|---|---|---|
| Protein | Fat | Protein | Fat | RC (g):Aqs Salt Solution (g) | RC (g) | a)Fat/ protein (%) |
| 0.59 | 0.001 | 0.56 | $6.10^{-4}$ | 1:26 | 20 | 0.07 |
| 0.50 | 0.004 | 0.54 | 0.002 | 1:13 | 1 000 | 0.36 |
| 0.57 | b.d.l | 0.56 | b.d.l. | 1:12.5 | 12 000 | <0.10 |

b.d.l: below detection limit.
a)Estimated with Eq (3).

Table 3: See Table2, but now with fat to protein ratio expressed as [1:X]

| Yield [g/g] | | extraction composition | | | | |
|---|---|---|---|---|---|---|
| protein | Fat | protein | fat | RC (g):asqs salt | RC(g) | Fat to protein [1 : X] |
| 0,59 | 0,001 | 0,56 | 6,00E-04 | 1:26 | 20 | 933 |
| 0,50 | 0,004 | 0,54 | 0,002 | 1:13 | 1000 | 270 |
| 0,57 | Bdl | 0,56 | b.d.l | 1 :12,5 | 12000 | > 1000 |

From these data it can be concluded that compared to extraction under mechanical mixing (see example 1), extraction with minimum shear (no mechanical mixing) leads to comparable or even higher protein extraction yields, with a relatively low fat extraction yield. Consequently, by using a gentle extraction an extract with a fat/protein ratio less than or equal to 0.5% is produced. Alternatively phrased (Table 3), the type of gentle extraction as used in example 3 results in a fat to protein ratio [1:X] of at least 1:200.

**Example 3 (prior art)**

This example describes the result of a conventional prior art (stirred vessel) aqueous extraction process.

Setup

A stirred vessel set-up consisting of a double-walled vessel with a volume of 4 L. The internal diameter of the vessel was 14.5 cm, which was equal to the liquid height when the vessel is filled. No baffles were installed. The agitation was controlled using an upper head stirrer equipped with an engine with a digital screen providing the actual stirrer speed in RPMs. The impeller was situated half way the liquid height (7 cm from the bottom). The used speed was 100 rpm. A waterbath was used to control the temperature. The applied solvent was demineralized water, mostly containing 30 mM (potassium) phosphate buffer. The pH was measured manually and set and adapted if needed with 4 M HCl and 4 M NaOH. After the aqueous solvent was added to the vessel and conditioned at the correct temperature and buffer strength, the rapeseed cake material was transferred to the vessel.

Analysis

Protein content was determined based on the Kjeldahl method, using a multiplication factor of 6.25 to determine the amount of protein (% (w/w)). Dry weight was determined by drying to a constant weight either using an infrared balance or overnight incubation in an oven at 105°C. Fat content in the samples was determined using the fatty acid methyl esters (FAME) analysis.

Results

In Table 4 the protein to fat ratios can be seen in time and at different temperatures. The results are also depicted in Figure 2. It is shown that in all cases the fat-protein ratio is lower than 11 and after more than 1 hour of extraction the ratios are all below 8.2 with an average of 5.7.

Table 4 Fat to protein ratio during time in a stirred vessel [1 : X], values determined by using equation (4)

| Time (min) | 10 degrees C | 30 degrees C | 50 degrees C |
|---|---|---|---|
| 15 | 11,0 | 8,0 | 6,3 |
| 30 | 9,3 | 8,1 | 6,5 |
| 45 | 9,5 | 6,6 | 5,8 |
| 60 | 8,2 | 5,7 | 5,0 |
| 90 | 7,8 | 5,2 | 5,6 |
| 120 | 7,1 | 4,6 | 4,6 |
| 180 | 5,9 | 4,4 | 4,3 |

**Example 4: prior art example**

Experimental setup

Mixing of the rapeseed cake and the extraction solvent (process water) to increase protein solubility). Extraction variables are the extraction temperature, pH, additives and mixing technique. The parameters are depicted in Table 5.

Table 5: Experimental parameters example 4

| Parameter | Run 1 | Run 2 |
|---|---|---|
| Temperature (°C) | 30 | 50 |
| pH | Adjusted to 7 | 6 |
| Rapeseed cake (kg) | 80 | 60 |
| Water (kg) | 450 | 300 |
| Solid to liquid weight ratio | 1 : 5.6 | 1 : 5 |
| Incubation time (hr) | 3 | 2 |
| Mixing | Propeller stirrer | Propeller stirrer |
| Mixer speed (rpm) | 950 | 600 |
| Power input (kW/hr) | 1.9 | 0.7 |
| Average shear rate (s$^{-1}$) | 158 | 100 |
| Tip speed (m/s) | 10 | 6 |
| Mixing time (s) | 2.7 | 2.8 |

Analysis

Protein contents have been determined with the Kjeldahl method (Flow injection analysis). The calculation factor used for converting nitrogen contents into protein contents was 6.25.
Dry matter analysis has been performed using a Mettler Toledo HG53 Halogen moisture analyzer (2 g sample on a disposable glas fibre filter, 105°C).
Fat has been determined via fatty acid methyl esters (FAME) analysis.

Run 1

In run 1, an overhead stirrer with a small propeller type of stirrer blade has been used to mix the (60 kg) rapeseed cake into the aqueous phase. The stirrer speed applied was very high (950 rpm).

Run 2

At lower mixing shear due to lower stirrer speed and high temperature to prevent microbial growth in the extraction, although fat levels in the extract were lower than in the previous experiment, the protein to fat ration has not been improved sufficiently.

Table 6 summarises all the data from example 4.

Table 6: data from example 4

| Parameter | Run 1 30°C, pH 7 | Run 2 50°C, pH 6 |
|---|---|---|
| Rapeseed cake : | | |
| Dry matter (kg) | 74 | 55 |
| Protein (kg) | 27,1 | 21 |
| Fat (kg) | 9,3 | 9,4 |
| Parameter Extract: | Run 1 | Run 2 |
| Weight extract | 380 | 201 |
| Dry matter (kg) | 33 | 16 |
| Dry matter (%) | 45 | 29 |
| Protein (kg) | 15,1 | 7,7 |
| Protein (%) | 46% | 48% |
| Fat (kg) | 7 | 2,3 |
| Fat (%) | 21% | 14% |
| Fat to protein ratio [1 :X] | 2,2 | 3,3 |

**Example 5: example not according to the invention (gentle extraction)**

Analysis

The samples were prepared by centrifugation of samples (10 min 4000 g), analysis of dry matter, fat and protein in supernatant. Protein content was determined based on the Kjeldahl method, using a multiplication factor of 6.25 to determine the amount of protein (% (w/w)). Dry weight was determined by drying to a constant weight either using an infrared balance or overnight incubation in an oven at 105°C. Fat content in the samples was determined using the fatty acid methyl esters (FAME) analysis.

Experimental setup

Testing effect of conveyance on fat and protein extraction. A 2 L volumetric cylinders (id 10 cm, height 35 cm) was used as a container for the liquid and rapeseed cake. 200 g sieved rapeseed cake was used with 1800 g of a 2% NaCl solution at room temperature. Two setups were used:

- Low frequency mixing: turning cylinder 180° and back every 30 minutes (total time: 150 minutes)
- High frequency mixing: turning cylinder 180° and back every 5 minutes (total time: 150 minutes)

This example does not include mechanic mixing and/or stirring.

Table 7: Protein to fat ratio after 150 min

| Column1 | Protein mg protein/gr sample | Protein on DWB % DWB | Fat % DWB | Ratio fat to protein [1:X] |
|---|---|---|---|---|
| Low frequency shaking | 19,9 | 35,1 | 2,1 | 16,9 |

(continued)

| Column1 | Protein<br>mg protein/gr sample | Protein on DWB<br>% DWB | Fat<br>% DWB | Ratio fat to protein [1:X] |
|---|---|---|---|---|
| **High frequency shaking** | **21,8** | **35** | **2,2** | 15,8 |
| Gentle extraction results in a fat to protein [1:X] ratio of at least 1:12. | | | | |

**Example 6: example according to the invention (gentle extraction; counter current)**

Method

Principle of the cascade or multistage extractor is mild (temperature range 10 - 60°C, pH 6 - 7) countercurrent extraction in a system allowing sufficient falling height for rapeseed particles to completely fall apart and allow maximal solubilisation of rapeseed proteins. Basically it involves dosing rapeseed cake on the top of a high cylindrically shaped vessel with the extracting liquid being fed from the bottom of the vessel. Rapeseed cake particles will hydrate and fall apart upon sedimenting through the cylinder. Depleted particles will sediment to the bottom of the vessel, where the concentrated solid fraction is being pumped away by a peristaltic pump. The saturated liquid will be collected from the top of the vessel due to an overflow mechanism.

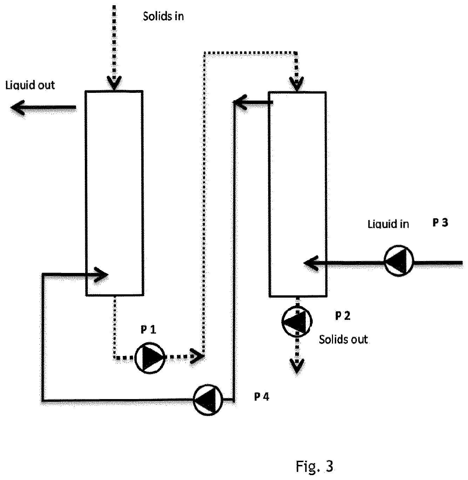

Description of Figure 3 Schematic extraction columns

Solids sedimenting to the bottom of the tube are being collected in the narrow adapter, and a bed of hydrated solids may be build up. The height of the bed is determined by the moment the solids removal pump is activated and the solid removal flow.

Via silicon tubing and solids removal pump 1 (P1) the solid fraction called "solids 1" is being transported into the top of the second tube. In tube 2 (stage 2), the solids will sediment as well, and will concentrate at the bottom of tube 2. Via another peristaltic pump (P2) and silicon tubing, the depleted solids called "solids 2" will be removed from the system.

The extracting solvent has been 2% NaCl solution , which is fed via a peristaltic pump (P3) to an inlet near the bottom of the 2nd tube. The flow rate of this pump determines the inlet flow of the NaCl solution. Prior to operating the tubes are being filled with 2% NaCl solution. The volume of the tubes is approx. 9 L. Extract at stage 2 (called "extract 1") is removed from the tube via an overflow construction at the top of tube 1. A septum is applied to limit solids into the extract flow. Extract 1 is collected in a smaller tube which serves as a buffer reservoir to prevent air from being introduced into tube 1 (in case removal of extract 1 is faster than the extract flow out of tube 2) and overflowing of tube 2 (in case the extract flow out of tube 2 is faster than the removal of extract 1 after the reservoir is filled (volume is approx. 1 L). Extract 1 is being transported into tube 2 via a fourth peristaltic pump (P4). The flow rate of this pump is being adjusted during runs to arrange partial filling of the reservoir.

Extract 1 is being fed into an inlet at the bottom of tube 2. Tube 2 has been equipped with an overflow system as well. The extract from tube 2 is being collected as product stream and is called "extract 2".

Materials

NaCl (GEF salt batch U-09143, SupraSel fine salt)
Rapeseed cake: Teutoburger Öhlmühle (TO), batch P071410
DIVOS 117, 7509021, lot no RSA09014
Gronfa 200 l vessel (04198)
Reko sieve bow (ZBM0304/001/W)

Balances:

Mettler Toledo PR8002 (F-BB-133)
Ohaus DA (F-BB-174)

Sartorius TE12000 (F-BB-197 and D-BL-080)

Temperature control:

Julabo F12 (F-BW-145) and F25 (D-BW-129) waterbath
Thermomix BU (T-BW-08)
Heat exchanger

Pumps:

Watson Marlow pump 520U (D-SP-045) and 520S (D-SP-054)
Watson Marlow pump 603U (I-SP-044) and 603S (I-SP-002)
Masterflex pump 7
Watson Marlow pump 501U (I-AE-041)
Mettler Toledo Halogen moisture analyzer HG63 (F-BL-014)
Seive: 1.4 mm
Centrifuge Harrier 18/80 (F-CF-064)
Retch Vibra hopper (F-OA-023)

Run

To test the productivity of the system, the NaCl solution flow rate has been increased to approx. 30 L/hr. To maintain the solid content of the extract, the rapeseed dosage rate has been increased to 500 g/hr. The solids removal pumps P1 and P2 have been activated at the start of the experiment, but the flow rates have been adjusted to allow the building up of a bed. The size of the bed was approx. 50% of the tube volume. At this bed size the flow rates of pumps P1 and P2 have been increased until the bed size was stable (approx. 6L/hr).
Rapeseed cake has been fed manually at the top of the left tube. Due to the very broad particle size distribution of the rapeseed cake (in $\mu$ range up to several mm), rapeseed cake has been sieved before use on a 1.4 mm sieve.
Dosing of rapeseed cake has been performed manually, with the help of a vibrating hopper in an attempt to arrange gradual dosing in time. The reservoir has been filled with a fixed amount of rapeseed cake every 10 minutes, and the vibrating frequency was adjusted to have this amount dosed in the 10 minutes interval. Due to the properties of the rapeseed cake, dosing could not be arranged more accurate than 5 - 10 minutes.

Analysis

Protein contents have been determined with the Kjeldahl method (Flow injection analysis). The calculation factor used for converting nitrogen contents into protein contents was 6.25.
Dry matter analysis has been performed using a Mettler Toledo HG53 Halogen moisture analyzer (2 g sample on a disposable glas fibre filter, 105°C).
Fat has been determined via fatty acid methyl esters (FAME) analysis.

Results

Table 8: data of example 6 (1 column volume is 9 liter)

| Extract volumes [column volumes] | Fat to protein ratio [1:X] |
|---|---|
| 3,07 | 22,5 |
| 3,6 | 22,7 |
| 4,12 | 20,7 |
| 5,68 | 21,9 |

(continued)

| Extract volumes [column volumes] | Fat to protein ratio [1:X] |
| --- | --- |
| 6,2 | 26,4 |
| 6,88 | 20,5 |

The use of recirculation percolation resulted in a fat to protein ratio [1:X] of at least 1:20. Repeated experiments resulted in fat to protein ratios [1:X] in the range of 1:20 to 1:30.

**Claims**

1. An aqueous method for producing from oil seed meal an intermediate aqueous protein solution having a fat to protein ratio of at least 1:12 comprising subjecting oil seed meal having an oil content of at least 8% on dry matter basis to gravity induced solid-liquid extraction wherein the solids essentially only move in a vertical way and optionally collecting the resulting intermediate aqueous protein solution, wherein said gravity induced solid-liquid extraction comprises percolation.

2. A method according to claim 1, wherein said oil seed meal is cold pressed oil seed meal.

3. A method according to any one of claims 1 to 2, wherein the oil seed meal is extracted with an aqueous solution by sprayed percolation, immersed percolation, solids dispersion or positive pressure percolation or recirculation percolation or multistage percolation or a combination thereof.

4. A method according to any one of claims 2 to 3, wherein said percolation is recirculation percolation.

5. A method according to any one of claims 1 to 4, wherein said extraction comprises an aqueous salt solution, preferably an aqueous salt solution comprising an ionic strength in the range of 0.10 to 0.8.

6. A method according to any one of claims 1 to 5, wherein said extraction is performed at a temperature in the range of 5-65°C.

**Patentansprüche**

1. Wässriges Verfahren zur Herstellung einer intermediären wässrigen Proteinlösung mit einem Fett-Protein-Verhältnis von mindestens 1:12 aus Ölsaatenmehl, bei dem man Ölsaatenmehl mit einem Ölgehalt von mindestens 8% in Bezug auf die Trockenmasse einer durch Schwerkraft erzeugten Fest-Flüssig-Extraktion unterzieht, wobei sich die Feststoffe im Wesentlichen nur vertikal bewegen, und gegebenenfalls Auffangen der entstandenen intermediären wässrigen Proteinlösung, wobei die durch Schwerkraft erzeugte Fest-Flüssig-Extraktion Perkolation umfasst.

2. Verfahren nach Anspruch 1, wobei es sich bei dem Ölsaatenmehl um kaltgepresstes Ölsaatenmehl handelt.

3. Verfahren nach einem der Ansprüche 1 bis 2, wobei das Ölsaatenmehl mit einer wässrigen Lösung durch Sprühperkolation, Immersperkolation, Feststoffdispersion oder Positivdruck-Perkolation oder Umwälzperkolation oder mehrstufige Perkolation oder eine Kombination davon extrahiert wird.

4. Verfahren nach einem der Ansprüche 2 bis 3, wobei es sich bei der Perkolation um die Umwälzperkolation handelt.

5. Verfahren nach einem der Ansprüche 1 bis 4, wobei die Extraktion eine wässrige Salzlösung, vorzugsweise eine wässrige Salzlösung mit einer Ionenstärke im Bereich von 0,10 bis 0,8, umfasst.

6. Verfahren nach einem der Ansprüche 1 bis 5, wobei die Extraktion bei einer Temperatur im Bereich von 5-65°C durchgeführt wird.

**Revendications**

1. Méthode aqueuse de production, à partir d'un tourteau de graines oléagineuses, d'une solution aqueuse de protéines intermédiaire ayant un rapport des matières grasses sur protéines d'au moins 1 : 12, comprenant la soumission d'un tourteau de graines oléagineuses ayant une teneur en huile d'au moins 8 % sur une base de matière sèche à une extraction solide-liquide induite par gravité, où les solides ne se déplacent essentiellement que de manière verticale et éventuellement la collecte de la solution aqueuse de protéines intermédiaire résultante, dans laquelle ladite extraction solide-liquide induite par gravité comprend une percolation.

2. Méthode selon la revendication 1, dans laquelle ledit tourteau de graines oléagineuses est un tourteau de graines oléagineuses pressé à froid.

3. Méthode selon l'une quelconque des revendications 1 à 2, dans laquelle le tourteau de graines oléagineuses est extrait avec une solution aqueuse par percolation pulvérisée, percolation immergée, dispersion de solides ou percolation sous pression positive ou percolation par recirculation ou percolation multi-étages ou une combinaison de celles-ci.

4. Méthode selon l'une quelconque des revendications 2 à 3, dans laquelle ladite percolation est une percolation par recirculation.

5. Méthode selon l'une quelconque des revendications 1 à 4, dans laquelle ladite extraction comprend une solution saline aqueuse, préférablement une solution saline aqueuse comprenant une force ionique dans la plage de 0,10 à 0,8.

6. Méthode selon l'une quelconque des revendications 1 à 5, dans laquelle ladite extraction est effectuée à une température dans la plage de 5-65°C.

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120252065 A
- US 6005076 A
- US 20100173064 A
- WO 2013013949 A
- WO 9527406 A
- GB 2461093 A
- GB 1502959 A
- WO 2012135955 A1, Rozenszain

### Non-patent literature cited in the description

- **ROSENTHAL et al.** *Enzyme and Microbial Technology,* 1996, vol. 19, 402-420
- **KHATTAB et al.** *LWT - Food Science and Technology,* 2009, vol. 42, 1119-1124
- **ROSENTHAL et al.** *Trans IChemE, Part C,* 1998, vol. 76, 224-230
- **LAWHON et al.** *Journal of Food Science,* 1981, vol. 46, 912-916
- **KLOCKEMAN et al.** *J. Agric. Food Chem,* 1997, vol. 45, 3867-3870
- **ROSENTHAL et al.** *Trans iChemE,* 1998, vol. 76
- **SHAHIDI F.** Canola and Rapeseed: Production, Chemistry, Nutrition and Processing Technology. Van Nostrand Reinhold, 1990
- General tests and assays, Appendix II. Food Chemical Codex. 1133-1134
- **KLOCKEMAN et al.** *Journal of Agricultural and Food Chemistry,* 1997, vol. 45, 3867-3870